# EUROPÄISCHE PATENTANMELDUNG

(11) **EP 0 727 407 A1**
(43) Veröffentlichungstag der Anmeldung: **21.08.1996**
(21) Anmeldenummer: 96102140.9
(22) Anmeldetag: 14.02.1996
(51) Int. Cl.: C07C 43/17, C07C 41/30

(54) **Mit verzweigten Perfluorethergruppen substituierte Diene, ihre Herstellung und Verwendung**

(30) Priorität: 20.02.1995 DE 19505665
(71) Anmelder: HOECHST AKTIENGESELLSCHAFT, 65926 Frankfurt am Main (DE)
(72) Erfinder: Von Werner, Konrad, Dr., D-84518 Garching (DE)

(57) **Zusammenfassung**

Durch Anlagerung von Iodiden der Formel 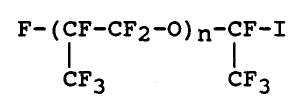 in der n eine Zahl von 1 bis 4 ist, an Diene der Formel

H₂C=CH-(CH₂)ₘ-CH=CH₂

in der m eine ganze Zahl von 4 bis 8 ist, und Abspaltung von Iodwasserstoff aus dem Anlagerungsprodukt, erhält man α,ω-Diene, die in α-Positionen mit verzweigten Perfluorethergruppen substituiert sind. Diese sind Comonomere für Polymerisationen oder Metathesen.

## Beschreibung

Die Erfindung bezieht sich auf α,ω-Diene, die in α-Position mit verzweigten Perfluorethergruppen substituiert sind, die Herstellung dieser Verbindungen durch Anlagerung der entsprechenden iodhaltigen Ether an die korrespondierenden α,ω-Diene, die so erhaltenen iodhaltigen Zwischenprodukte und deren Überführung in die Diene sowie die Verwendung der genannten Produkte.

Die Erfindung betrifft zunächst die neuen Diene der Formel (1) 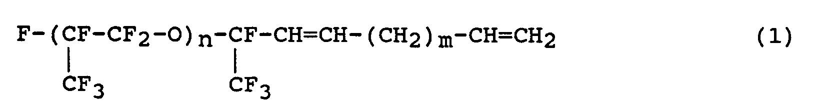 in der m eine ganze Zahl von 4 bis 8 und n eine Zahl von 1 bis 4 ist. Die Erfindung betrifft somit die reinen Diene, in denen n für eine ganze Zahl steht, aber auch Gemische aus Verbindungen der Formel (1), in denen n unterschiedliche Bedeutungen im Bereich von 1 bis 4 aufweist.

Die Erfindung betrifft weiterhin Verbindungen der Formel (2) 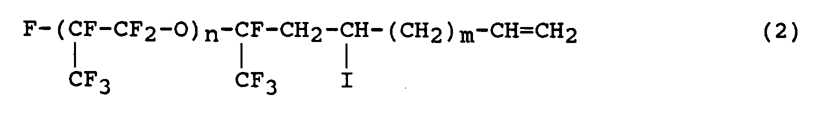 in der m und n die vorstehend genannten Bedeutungen haben. Diese Verbindungen stellen Zwischenprodukte für die Herstellung von Verbindungen der Formel (1) dar, aber auch für die entsprechenden ω-Monoene.

Die Erfindung betrifft weiterhin ein Verfahren zur Herstellung von Verbindungen der Formel (1), bei dem man Verbindungen der Formel (3) 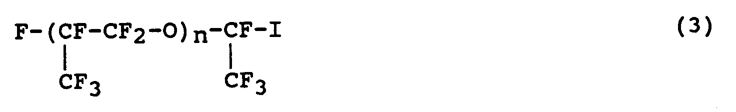 an ein Dien der Formel (4)

H₂C=CH-(CH₂)ₘ-CH=CH₂ (4)

wobei n und m die vorstehend genannten Bedeutungen haben, anlagert und aus der so erhaltenen Verbindung der Formel (2) Iodwasserstoff abspaltet.

Die Erfindung betrifft weiterhin die Verwendung von Verbindungen der Formel (1) als Comonomere für Polymerisations- und Metathesereaktionen.

Weitere Aspekte der Erfindung und ihre bevorzugten Ausgestaltungen werden im folgenden näher erläutert.

Die Herstellung von Verbindungen der Formel (2) kann entsprechend der Anlagerung von Perfluoralkyliodiden an Alkene erfolgen. Üblicherweise wird hierfür ein radikalisch zerfallender Starter eingesetzt, meist eine organische Peroxo- und Diazoverbindung. Es wurde gefunden, daß sich für die erfindungsgemäße Anlagerung des Diens der Formel (4) an das Iodid der Formel (3) als Initiator das Diazo-bis(1,1-dimethylpropan) besonders gut eignet.

Vorteilhaft erfolgt diese erfindungsgemäße Addition in einem Temperaturbereich von 70 bis 130 °C, insbesondere bei 90 bis 110 °C.

Das Dien der Formel (4) wird zweckmäßig im Überschuß, vorteilhaft 2 bis 4 mol Dien der Formel (4) pro mol des Iodids der Formel (3), eingesetzt. Das überschüssige Dien wird nach Beendigung der Additionsreaktion zurückgewonnen, zweckmäßig durch Destillation.

In einer bevorzugten Ausführung der erfindungsgemäßen Additionsreaktion wird das Dien im Überschuß vorgelegt und das Iodid - absatzweise oder kontinuierlich - zudosiert. Der Initiator wird ebenfalls kontinuierlich oder absatzweise dem Reaktionsgemisch zugegeben, vorteilhaft in Form einer Lösung im Iodid.

Im allgemeinen wird in einer Schutzgasatmosphäre, beispielsweise Kohlendioxid oder Stickstoff, gearbeitet.

Die Menge des Initiators beträgt zweckmäßig 0,5 bis 10 Mol-%, insbesondere 1 bis 5 Mol-%, bezogen auf das Iodid der Formel (3).

Nach Beendigung der Umsetzung wird das überschüssige Dien abgetrennt, vorteilhaft durch Destillation im Vakuum. Der mehr oder weniger flüssige Rückstand besteht hauptsächlich aus der Verbindung der Formel (2). Dieser Rückstand wird in einem polaren, wassermischbaren Lösemittel, wie zum Beispiel einem Mono- oder Dimethylether von Di- oder Triethylenglykol, vorteilhaft einem niederen Alkanol, insbesondere Ethanol, gelöst. Zu dieser Lösung gibt man unter Rühren eine Base, vorteilhaft Natrium- oder Kaliumhydroxid oder -carbonat, vorteilhaft in Form von mehreren Portionen oder kontinuierlich. In einer zweckmäßigen Ausgestaltung der Erfindung wird die Base in dem gleichen Lösemittel gelöst oder dispergiert, das für die Lösung des Rückstandes eingesetzt wurde.

Vorteilhaft wird das Lösemittel für den Rückstand und gegebenenfalls die Base in weitgehend wasserfreier Form eingesetzt. In diesem Falle scheidet sich das Alkalimetalliodid weitgehend in Form eines Niederschlags ab.

Die Umsetzung mit der Base kann in einem Temperaturbereich von 0 bis 120 °C, vorteilhaft 10 bis 50 °C, insbesondere bei Raumtemperatur, erfolgen. Falls erforderlich, kann man das Reaktionsgemisch noch unter erhöhter Temperatur, zweckmäßig bei Rückflußtemperatur, nachrühren.

Zur Abtrennung des Alkalimetalliodids wird der Reaktionsansatz zweckmäßig mit Wasser versetzt, wobei sich das Alkalimetalliodid in der sich bildenden oberen Phase löst. Nach Abtrennung dieser wäßrigen Phase wird diese mit einem geeigneten, nicht mit Wasser mischbaren Lösemittel, vorteilhaft mit einem Lösemittel hoher Dichte, ein- oder mehrmals ausgeschüttelt. Die gegebenenfalls vereinigten Unterphasen können hierauf nochmals mit Wasser ausgeschüttelt werden, worauf das Reaktionsprodukt vom Lösemittel abgetrennt wird, zweckmäßig durch Destillation.

Die Produkte der Formel (1) fallen überwiegend als trans-Isomere (E-Konformation) an. Die Produkte zeichnen sich dadurch aus, daß sie über einen weiten Temperaturbereich flüssig sind. Diese Eigenschaft ist für die weitere Verarbeitung vorteilhaft: So kann man beispielsweise bei der Copolymerisation mit anderen Olefinen oder bei einer Metathesereaktion auf den Zusatz von Lösebeziehungsweise Verdünnungsmitteln verzichten.

Werden die Verbindungen der Formel (1) bei der Metathese entsprechend der US-A 5 146 017 eingesetzt, so reagiert nur die ω-ständige Doppelbindung, also nicht die Doppelbindung, an die die fluorierte Ethergruppe gebunden ist. Man erhält somit Metatheseprodukte, die noch diese letztgenannte Doppelbindung enthalten. Falls gewünscht, kann diese erhaltene Doppelbindung anschließend in eine gesättigte Verbindung überführt werden, beispielsweise durch Hydrierung. Auf diese Weise läßt sich die Stabilität der Endprodukte, beispielsweise gegen UV-Strahlung und erhöhte Temperatur, erhöhen. Zu den entsprechenden gesättigten Produkten kann man auch aus den Monoenen gelangen, die man durch Entfernung des Iods aus den Verbindungen der Formel (2) erhält. Diese Substitution des Iodatoms durch Wasserstoff ist in an sich bekannter Weise, beispielsweise mit Zink und Säure, möglich.

Die Metathesereaktion kann auch als Cometathese mit α-Olefinen oder Cycloolefinen stattfinden. Bei allen Metathesereaktionen und auch bei der Copolymerisation mit Olefinen erhält man Produkte, die gute oleophobe und hydrophobe Eigenschaften aufweisen und die sich als Gleitmittel eignen.

Die erfindungsgemäßen Produkte können auch analog den Additionsprodukten von Perfluoralkyliodiden an Diene eingesetzt werden. So ist beispielsweise aus C & EN, September 6, 1993, Seite 33 bekannt, n-Perfluordecyliodid mit 1,7-Octadien umzusetzen und das Anlagerungsprodukt in ein α,ω-perfluoralkylsubstituiertes Polyethylen zu überführen.

In den folgenden Beispielen wird die Erfindung näher erläutert. Prozentangaben beziehen sich auf das Gewicht, sofern keine anderen Angaben gemacht sind.

Die Ausgangsmaterialien der Formel (3) und ihre Herstellung sind aus der EP-B 295 582 bekannt.

### Beispiel 1

Die Apparatur besteht aus einem 250-ml-Vierhalskolben mit Rührer, Tropftrichter, Rückflußkühler und Thermometer. Der Kühler ist mit einem Ventil zum Druckausgleich verschlossen. Der Tropftrichter wird befüllt mit einer Lösung von 0,55 g Diazo-bis(1,1-dimethylpropan), gelöst in dem Iodid der Formel 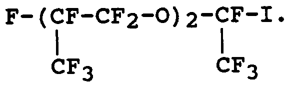 66,2 g (0,6 mol) 1,7-Octadien werden im Rührkolben vorgelegt. Die Apparatur wird mit Stickstoff gespült und verschlossen. Der Kolben wird auf 100 °C Innentemperatur beheizt und die Lösung des Initiators in dem Iodid im Laufe von 2 Stunden bei dieser Temperatur zugetropft. Man rührt noch 30 Minuten nach und gibt dann 0,14 g des Initiators, gelöst im Iodid, zu, so daß insgesamt 111,8 g (0,2 mol) des Iodids eingesetzt werden. Die Mischung wird 2 Stunden bei 100 bis 110 °C nachgerührt. Nach Abkühlen auf etwa 70 °C wird das überschüssige Octadien bei 15 mbar und einer Sumpftemperatur von maximal 90 °C abdestilliert.

Der Rückstand wird in 1 l Ethanol gelöst. Zu dieser Lösung gibt man 14,84 g festes Kaliumhydroxid (85%ig, entsprechend 0,24 mol) in fünf Portionen bei Raumtemperatur innerhalb von 3 Minuten zu. Es bildet sich ein Niederschlag von Kaliumiodid. Zur Vervollständigung der Reaktion rührt man noch 1 Stunde unter Rückfluß nach.

Nach Abkühlen auf Raumtemperatur wird 1 l entsalztes Wasser zugegeben. Die sich bildende Unterphase wird abgetrennt und die obere Phase mit 100 ml 1,1,2-Trichlor-trifluorethan ausgeschüttelt. Die vereinigten Unterphasen werden mit 200 ml Wasser geschüttelt, die organische Phase abgetrennt und destilliert. Nach Entfernen des Trichlor-trifluorethans bei Normaldruck wird der Rückstand bei 14 mbar über eine Kolonne fraktioniert. Man erhält als Hauptlauf 78,9 g einer farblosen Flüssigkeit vom Siedepunkt 99,5 bis 102 °C. Das Produkt wird durch folgende NMR-Daten charakterisiert:

CDCl₃-Lösung, ¹H-NMR bei 300,13 MHz (Standard TMS), ¹⁹F-NMR bei 282,39 MHz (Standard Trifluoressigsäure).

¹H-NMR:

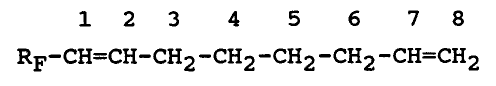

### E-Isomeres:

- 1: 5,64 ppm (t, 15,5 Hz)
- 2: 6,44 ppm (d, t, 15,6 Hz, 6,9 Hz)
- 3: 2,21 ppm (m)
- 4 5: 1,45 ppm (m)
- 6: 2,06 ppm (m)
- 7: 5,78 ppm (m)
- 8: 4,97 ppm (3 Signale)

### Z-Isomeres:

- 1: 5,5 ppm (d, d)
- 2: 6,12 ppm (m)
E : Z = 14,6 : 1

### ¹⁹F-NMR:

- E-Isomeres:: α-CF: 51,5 ppm, β-CF:67,6 ppm
- Z-Isomeres:: α-CF: 47,1 ppm, β-CF:67,3 ppm
E : Z = 14,5 : 1

### Beispiele 2 bis 4

Die Umsetzung erfolgt analog Beispiel 1. Die Ergebnisse zeigt die folgende Tabelle, wobei Diene der Formel (1) mit m = 4 erhalten werden, in denen n = 1, 3 und 4 ist.

**Tabelle**

| Beispiel | n | Ausbeute [%] | E/Z-Verhältnis | Kochpunkt [°C/mbar] |
|---|---|---|---|---|
| 2 | 1 | 37,9 | 9,5 : 1 | 66 bis 68/14 bis 15 |
| 3 | 3 | 56,5 | 16,0 : 1 | 94,5 bis 95,5/2 |
| 4 | 4 | 57,0 | 14,9 : 1 | 99 bis 101/0,05 |

### Beispiel 5

Analog zu Beispiel 1 wird ein Dien der Formel (1) mit n = 2 und m = 6 hergestellt. Das Produkt ist eine farblose Flüssigkeit mit Kochpunkt 112 bis 115 °C/14 mbar (Ausbeute 65 %, E/Z-Verhältnis 14,5 : 1).

## Patentansprüche

1. Verbindungen der Formel (1) 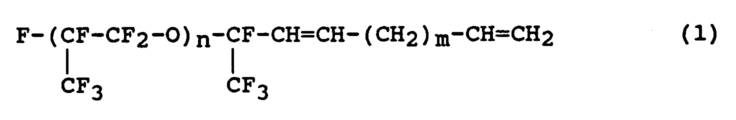 in der m eine ganze Zahl von 4 bis 8 und n eine Zahl von 1 bis 4 ist.

2. Verbindungen der Formel (2) 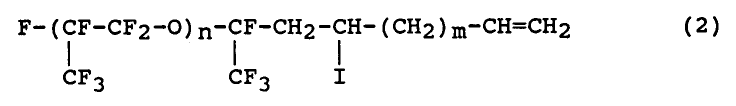 in der m und n die in Anspruch 1 genannten Bedeutungen haben.

3. Verfahren zur Herstellung von Verbindungen der Formel (1) 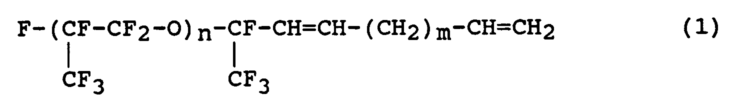 in der m eine ganze Zahl von 4 bis 8 und n eine Zahl von 1 bis 4 ist, dadurch gekennzeichnet, daß man Verbindungen der Formel (3) 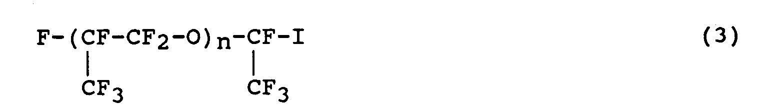 an ein Dien der Formel (4)
H₂C=CH-(CH₂)ₘ-CH=CH₂ (4)
anlagert und aus der so erhaltenen Verbindung der Formel (2) 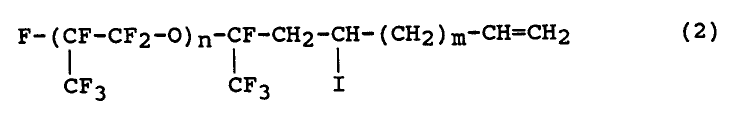 Iodwasserstoff abspaltet, wobei m und n jeweils die genannten Bedeutungen haben.

4. Verfahren nach Anspruch 3, dadurch gekennzeichnet, daß die Anlagerung mit Diazo-bis(1,1-dimethylpropan) als Initiator erfolgt.

5. Verwendung von Verbindungen der Formel (2) zur Herstellung von Verbindungen der Formel (1).

6. Verwendung von Verbindungen der Formel (1) als Comonomere.
